(19) **Europäisches Patentamt**
**European Patent Office**
**Office européen des brevets**

(11) **EP 2 829 876 B1**

(12) **EUROPEAN PATENT SPECIFICATION**

(45) Date of publication and mention
of the grant of the patent:
**30.08.2017 Bulletin 2017/35**

(51) Int Cl.:
*G01N 33/46* *(2006.01)*     *G01N 22/00* *(2006.01)*

(21) Application number: **14178236.7**

(22) Date of filing: **23.07.2014**

(54) **Method and apparatus for estimating the projection on a reference plane of the direction of extension of the fibres of a portion of a wooden plank**

Verfahren und Vorrichtung zur Abschätzung der Projektion auf eine Referenzebene der Richtung der Fasern eines Teils eines Holzbrettes

Méthode et dispositif pour estimer la projection sur un plan de référence de la direction des fibres d'une partie d'une planche de bois

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.07.2013 IT BZ20130037**

(43) Date of publication of application:
**28.01.2015 Bulletin 2015/05**

(73) Proprietor: **MICROTEC S.r.l.**
**39042 Bressanone (Bolzano) (IT)**

(72) Inventors:
• **Denzler, Julia**
**80995 Muenchen (IT)**
• **Koppensteiner, Johannes**
**3500 Krems (AT)**
• **Weidenhiller, Andreas**
**1130 Wien (AT)**

• **Arthaber, Holger**
**1060 Wien (AT)**
• **Leder, Norbert**
**2285 Leopoldsdorf I.M (AT)**

(74) Representative: **Ponchiroli, Simone**
**Ruffini Ponchiroli e Associati S.r.l.**
**Via Caprera, 6**
**37126 Verona (IT)**

(56) References cited:
**US-A- 4 514 680     US-A1- 2003 222 657**

• **BOGOSANOVIC M ET AL: "Microwave nondestructive testing of wood anisotropy and scatter", January 2013 (2013-01), IEEE SENSORS JOURNAL 2013 INSTITUTE OF ELECTRICAL AND ELECTRONICS ENGINEERS INC. USA, VOL. 13, NR. 1, PAGE(S) 306 - 313, XP002717863, * the whole document ***

## Description

This invention relates to a method and an apparatus for estimating the projection on a reference plane of the direction of extension of the fibres of a portion of a wooden plank, as well as a device which constitutes the operating core of the apparatus. It should be noticed that the expression "estimating the projection" in the context of this invention refers to an estimate of the orientation of the projection itself.

This invention, in particular, is intended for the sector of apparatuses for grading timber which are currently used to determine, for example, the mechanical resistance or the modulus of elasticity of cut timber.

Therefore, the application sector is that of timber used for structural purposes, for example for the construction of buildings. In this context, the orientation of the fibres of the wood in a single piece of timber is particularly important. In fact, the resistance of the timber to bending, tension and compression may be reduced to as little as one tenth of the value if the orientation of the fibres passes from an alignment with the nominal direction envisaged (angle 0°) to an alignment perpendicular with said nominal direction (angle 90°). Timber with orientation of the fibres which is too far from the nominal direction is not suitable for use in construction. It should be noticed that the nominal direction of the fibres of a wooden plank is normally the direction parallel with the two pairs of main faces of the plank itself.

From a terminological viewpoint, in the timber sector (and hereinafter) reference is made to the direction of the grain to indicate the orientation of the fibres in space, and to deviation of the grain to indicate any deviation of the orientation of the fibres relative to the nominal direction.

It should be noticed that deviations of the grain may either affect the entire length of a piece of wood (global deviations) or may be present only locally (local deviations) depending on what caused them.

In general, global deviations of the grain are in fact caused by growth of the trunk as a whole which may be curved, with fibres in a spiral shape or tapered (especially towards the tip). However, in general global deviations are of limited extent (at most several tenths of a degree).

In contrast, local deviations may be extremely significant (up to 90° relative to the nominal direction). These are usually caused by anything that constituted an irregularity in the growth of the trunk, such as knots and breaks/wounds.

The easiest way to assess deviations of the grain has always been an examination of the surface of the timber carried out by an operator and intended to identify the trend of the fibres at the surface.

Obviously, such an examination method, as well as not allowing any assessment of the inside of the plank, is relatively time-consuming, which is not compatible with the production rates now required in the sector.

Therefore, as time passed, techniques were studied which could allow automated assessment of the deviations of the grain.

In particular, techniques were developed which were based either on an examination of only the surface of the timber (therefore, the concepts being equivalent to visual examination by an operator) or techniques which also take into consideration the inside of the piece of timber.

The latter include methods based on the transmission of microwaves through the wood.

In particular, in the prior art for a couple of decades now microwaves have been transmitted through wood to determine the angle formed by the fibres of the wood in the plane perpendicular to the direction of propagation of the microwaves (which is advantageously selected perpendicular to a flat face of the timber). In other words, what is assessed is the angle formed relative to the nominal direction of the fibres, by the projection of the actual fibres on the face through which the microwaves pass. This angle is usually called the horizontal angle (even if sometimes also the slope angle). It should be noticed that the horizontal angle coincides with the deviation of the grain only if the deviation of the grain occurs parallel with the face of the plank. In all other cases the horizontal angle only represents the projection, on said face, of the deviation of the grain.

The known methods for determining the horizontal angle are covered in many publications including:

- Bogosanovic M, Anbuky A.A, Emms G.W (2009): Microwave measurement of wood in principal directions, Sensors, 2009 IEEE, pp. 252-256;
- Bogosanovic M, Anbuky A.A, Emms G.W (2010): Overview and comparison of microwave noncontact wood measurement techniques, Journal of Wood Science 56 (5), pp. 357-365;
- Bogosanovic M, Anbuky A.A, Emms G.W (2011): Microwave measurement of wood anisotropy, Sensors Applications Symposium (SAS), 2011 IEEE, pp. 262-267;
- Heikilla S, Jakkula P, Tiuri M (1982) Microwave methods for strength grading of timber and for automatic edging of boards, Proceedings of the 12th European Microwave Conference. Helsinki, pp 599-603;
- James W.L, Yen YH, King R.J (1985): A microwave method for measuring moisture content, density and grain angle of wood, Forest Products Laboratory, Research Note FPL-0250;
- Leicester R.H, Seath C.A (1996) Application of microwave scanners for stress grading, Conference Proceedings - International Wood Engineering Conference 2: 435-440;
- Malik S.A, Ghodogoankar D.K, Hambaly A.M, Majid W.M, Nuruddin M.F (2005) Measurement of wood grain angle using free-space microwave system in 8-12GHz frequency range, Proceedings of the Asian Conference on Sensors and the International Conference on New Techniques in Pharmaceutical and Biomedical Research. Kuala Lumpur, pp 213-218;

- Martin P, Collet R; Barthelemy P, Roussy G (1987): Evaluation of wood characteristics: Internal scanning of the material by microwaves, Wood Science and Technology 21/4, pp. 361-371;

- Schajer G.S, Orhan F.B (2005): Microwave non-destructive testing of wood and similar orthotropic materials, Subsurface Sensing Technologies and Applications 6, pp. 293-313; and

- Shen J, Schajer G.S, Parker R (1994): Theory and practice in measuring wood grain angle using microwaves, IEEE Trans Instrum. Meas. 43: 803-809.

In particular, Bogosanovic M, Anbuky A.A, Emms G.W (2010): Overview and comparison of microwave noncontact wood measurement technique, Journal of Wood Science 56 (5), pp. 357-365, provides a complete review of them.

Said methods are also the subject matter of several patents, including: CA 2,046,895, US 6,842,010, US 4,500,835 and US 6,859,046.

However, all of the prior art methods for determining the horizontal angle can only work well if the orientation of the fibres is substantially the same over the entire thickness of the plank through which the microwaves pass. In fact, the information which can be obtained from the microwaves exiting represents the sum of the effects of all of the individual fibres passed through (basically one result for the entire thickness).

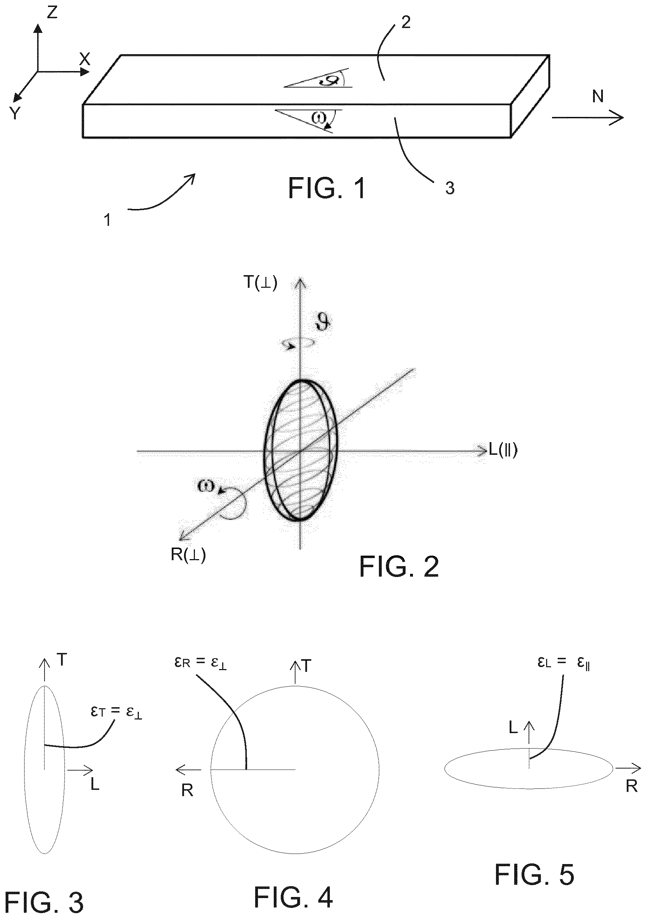

In any case, whilst the use of microwaves to determine the horizontal angle is now well known, there are still no prior art methods reliable enough to use microwaves to determine or at least estimate the vertical angle or dive angle. Said definition can usually be used to identify both the absolute angle formed by the fibres of wood relative to a plane parallel with the face observed (angle formed by fibres entering the face - that is angle with respect to plane XY in Fig. 1), and the projection of said absolute angle on a plane perpendicular to the face observed (that is on plane XZ in Figure 1).

At present, the only solution that has been proposed for determining the dive angle is described in the above-mentioned patent US 6,842,010. Said patent is based on the consideration that what can be defined as the dive angle (understood to be a projection) when a first face of the plank is observed, may be defined as the horizontal angle when a second face of the plank is observed which is perpendicular to the first face. The patent therefore suggests that the dive angle is determined by rotating the plank or the device through 90° about the nominal direction of the fibres and taking the measurement of the horizontal angle relative to the second face. The three-dimensional direction of the fibres is then calculated as the composition of the two slope angles determined on the two faces.

However, it was seen that this solution can only be effectively applied with a very limited range of plank dimensions. In fact, there may be problems both if a transversal dimension is too large and if it is too small. In fact, in the former case, on one hand there is a maximum thickness of the wood beyond which it is not possible to obtain the transmission of microwaves. On the other hand the greater the thickness passed through, the higher the risk that the wood is not substantially uniform, which would make any assessment useless (in fact, it should be remembered that the microwaves received are affected by the sum of effects of all of the individual fibres passed through). In contrast, in the latter case the frequency/wavelength of the microwaves means that, if the dimension of the plank transversally to the direction of incidence is too small, diffraction of the waves prevails over the transmission, therefore making any assessment impossible.

A solution similar to the one of US 6,842,010 is disclosed in US 2003/222657 where, in accordance with the content of paragraph [0039] dive angle is calculated as geometric combination of two horizontal angles determined according to two directions of observation perpendicular one to the other. US 2003/222657, in fact, teaches to directly estimate the projection of the fibre's angle only on a reference plane perpendicular to the direction of observation/irradiation; in accordance with the teaching of US 2003/222657 is by contrast impossible to make the estimate by examining a face of the plank which is orthogonal to the reference plane.

In light of that, considering the fact that wooden planks usually have a reduced thickness which is significantly less than the width, it seems obvious how in many cases it is impossible to determine the horizontal angle at the smaller face and it would therefore be preferable to be able to also assess the dive angle by examining the plank perpendicularly to its width, as is commonly done for the slope angle.

In this context, the technical purpose which forms the basis of this invention is to provide a method and an apparatus for estimating the projection on a reference plane of the direction of extension of the fibres of a portion of a wooden plank which overcomes the above-mentioned disadvantages.

In particular, the technical purpose of this invention is to provide a method and an apparatus for estimating the projection on a reference plane of the direction of extension of the fibres of a portion of a wooden plank which allows the estimate to be made by examining a face of the plank which is orthogonal to the reference plane.

The technical purpose specified and the claims indicated are substantially achieved by a method and an apparatus for estimating the projection on a reference plane of the direction of extension of the fibres of a portion of a wooden plank as described in the appended claims.

Further features and the advantages of this invention are more apparent in the detailed description of several preferred, non-limiting embodiments of a method and an apparatus for estimating the projection on a reference plane of the direction of extension of the fibres of a portion of a wooden plank, with reference to the accompanying drawings, in which:

- Figure 1 shows a wooden plank with the horizontal angle υ and the dive angle ω highlighted (both understood to be projections on the respective faces of the plank in accordance with what is indicated above);
- Figure 2 shows the ellipsoid illustrating the dependence on the orientation in space of the dielectric behaviour of a wood fibre;
- Figure 3 is a schematic side view in the LT plane of the ellipsoid of Figure 2;
- Figure 4 is a schematic front view in the RT plane of the ellipsoid of Figure 2;
- Figure 5 is a schematic top view in the LR plane of the ellipsoid of Figure 2;
- Figure 6 is a schematic side view of a first embodiment of the present invention;
- Figure 7 is a schematic side view of an apparatus made according to a second embodiment of this invention;
- Figure 8 is a front view of an alternative embodiment of the apparatus of Figure 7 with some parts cut away to better illustrate others; and
- Figure 9 shows the combined effect of three emissions/readings relating to the same portion of a wooden plank.

To make the explanation of this invention clearer, hereinafter there is first an explanation of the principle of physics on which it is based, then of the apparatus made according to this invention and finally of the method.

As is known, the dielectric behaviour of wood is anisotropic. Consequently, it is possible to represent it using a characteristic surface, as is normally the case for any property of anisotropic materials. In the case of the dielectric behaviour of wood, given the dielectric tensor $\varepsilon$ which describes the interaction of the wood with an external electric field depending on its orientation, its variation in space may be represented by the surface of an ellipsoid which represents the locus of the vertices of the tensor $\varepsilon$. The ellipsoid is positioned with its semi-axes oriented depending on the orientation of the wood fibres.

In particular, it is possible to identify a longitudinal semi-axis (extending along direction L in Figure 2) which is parallel with the direction of the wood fibres, a radial semi-axis (extending along direction R in Figure 2) and a tangential semi-axis (extending along direction T in Figure 2); the respective lengths being:

$$\frac{1}{\sqrt{\varepsilon_L}} \; ; \; \frac{1}{\sqrt{\varepsilon_R}} \; ; \; \frac{1}{\sqrt{\varepsilon_T}}$$

where $\varepsilon_L$, $\varepsilon_R$, $\varepsilon_T$, represent the dielectric constants in the three directions.

However, the radial and tangential dielectric properties are very similar and may therefore be considered substantially equal and defined overall as properties which are perpendicular ($\perp$) to the direction of the grain. In line with that, the longitudinal property may also be defined as a property which is parallel ($\parallel$) with the direction of the grain. Therefore, the respective dielectric constants will be $\varepsilon_\perp$ and $\varepsilon_\parallel$.

As shown in Figures 2 to 5, the ellipsoid is oriented depending on the spatial orientation of the wood fibres with the shortest semi-axis, corresponding to $\varepsilon_\parallel$, aligned with the direction of the grain L. The ellipsoid is therefore a sort of disk whose axis of symmetry coincides with the direction of extension of the fibres L.

The first idea which forms the basis of this invention is that to determine the orientation of the fibres it is sufficient to determine the orientation of the ellipsoid which represents the dielectric tensor $\varepsilon$. In fact, the direction of the smallest semi-axis coincides with the direction of the fibres L.

The second idea which forms the basis of this invention is that to determine the three-dimensional orientation of the fibres of the wood, the problem may be broken down by separately determining two projections of the direction of the grain L on two planes which are perpendicular, as shown in Figure 1 where the angle υ represents the projection on a first flat face 2 (parallel to plane XY in Figure 1) of the plank 1, and the angle ω represents the projection on a second flat face 3 (parallel to plane XZ in Figure 1) of the plank 1 which is orthogonal to the first 2. However, the angle υ corresponds to the horizontal angle and may therefore be determined using the prior art methods referred to above.

Consequently, to determine the three-dimensional orientation of the fibres it is sufficient to find a method for estimating the angle ω.

The third idea which forms the basis of this invention is that the interaction between a beam of incident microwaves and a fibre of the wood, each having its own orientation in space, may be considered as a sort of projection of the ellipsoid. Conceptually, it is a situation similar to that of an object which produces different shadows if lit from different viewpoints. But in this case, the "shadow" is not the projection of the entire ellipsoid, but rather the projection of the ellipse which is obtained from the intersection of the ellipsoid with a plane passing through its centre and perpendicular to the direction of "lighting". Moreover, the projection is shown as a variation of the intensity and a phase shift of the incident wave.

In light of those conditions, first what is usually a three-dimensional problem can be reduced to a two-dimensional problem by reasoning only with reference to the projections of the ellipsoid and of the dive angle on a reference plane XZ which is perpendicular to the first face 2 of the plank 1 (the one against which the microwaves are directed). To do that, it can be sufficient to irradiate the wood from a single side for example the reference plane XZ with microwaves of defined polarization. In fact, in this way, rather than considering the entire ellipsoid it is possible to consider a single two-dimensional ellipse, the one identified by the intersection of the ellipsoid with a plane parallel with the reference plane XZ and passing through the centre of the ellipsoid. In the invention the microwaves are polarized exclusively in a plane parallel with the reference plane XZ.

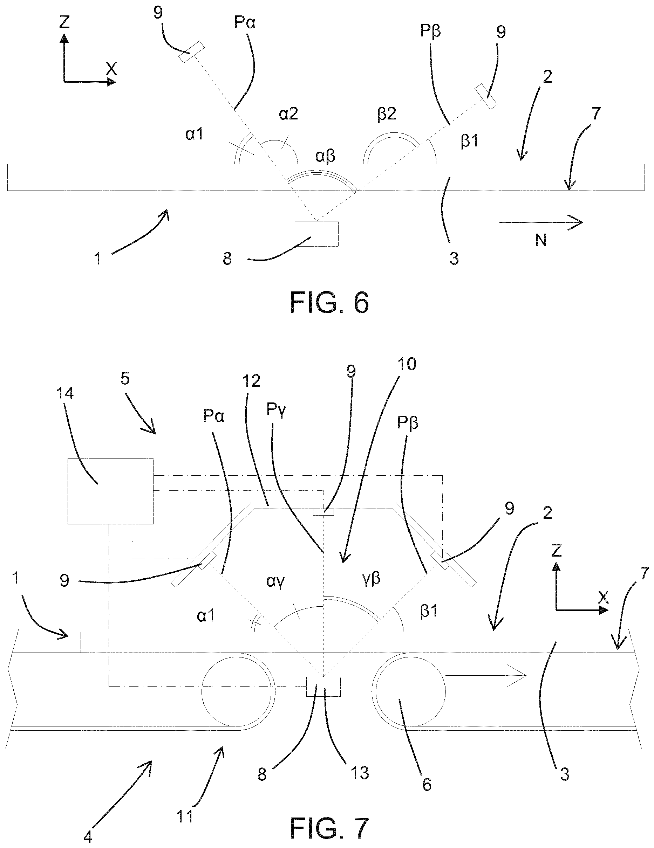

Secondly, as shown in Figure 6, it is usually sufficient to irradiate with polarized microwaves the wood whose direction of grain L is to be determined in two directions $P\alpha$, $P\beta$ which are incident on the first face 2 and which lie in a plane perpendicular to the first face 2 (and therefore parallel with the reference plane XZ). The two directions $P\alpha$, $P\beta$ must also be set at a sufficient angle ($\alpha\beta$) to each other to provide two sufficiently separate projections of the relevant ellipsoid. Therefore, from the two projections it is possible to determine the tensors $\varepsilon$ which are perpendicular to the directions of irradiation $P\alpha$, $P\beta$, from which the ellipse can be calculated which corresponds to the intersection of the ellipsoid with the vertical plane passing through its centre and parallel with the reference plane XZ. Once the ellipse is known, it is possible to determine the semi-axes, and the angle formed by the smallest semi-axis of the ellipse obtained in that way corresponds to the dive angle $\omega$. In this greatly simplified model it is therefore sufficient to know the two projections in order to estimate the angle $\omega$.

However, if the wood is irradiated according to a direction of incidence which is set at an angle to the first face 2 (that is to say, with angle of incidence $\neq 90°$) it must be remembered that the microwaves are subject to refraction as they pass from the air to the wood and again when they pass back from wood to air. In fact, the angle with which the microwaves propagate in the wood is not the angle of incidence, but an angle $\varphi$ (measured relative to a direction perpendicular to the first face 2) which depends also on density and degree of moisture of the wood, as known to the skilled person. Consequently, what is detected is not the dielectric response of the wood in the direction identified by the angle of incidence, but that in the direction identified by the angle $\varphi$.

Moving on to the description of the apparatus 4 for implementing this invention, it is illustrated as a whole in Figure 7.

As indicated, the apparatus 4 allows an estimate of the projection on a reference plane XZ of the direction of extension of the fibres L of a portion of a wooden plank 1.

As shown in Figure 1, the wooden plank 1 has a nominal direction of the fibres N which is parallel with a main axis of extension of it and a first face 2 which is also parallel with the main axis of extension (advantageously, the first face 2 is always one of the two opposite faces having the largest surface area).

The core of the apparatus 4 is a device 5 for examining a wooden plank 1 which first comprises supporting means 6 for the plank 1 which define both a lying plane 7 (parallel to plane XY) on which, during operation, the first face 2 of the plank 1 or the face diametrically opposed to it lies, and a longitudinal axis which is parallel with the lying plane 7 (parallel with the sheet in Figure 7). In use, the plank 1 is placed on the supporting means 6 with the nominal direction of the fibres N parallel with the longitudinal axis.

The device 5 also comprises both means for emitting 8 polarized microwave beams which are positioned on a first side of the lying plane 7, and means for detecting 9 microwaves which are positioned on a second side of the lying plane 7 which is opposite to the first side. In use, the detecting means 9 allow detection of the residual part of the polarized microwave beams emitted by the emitting means 8 after they have passed through the plank 1. For that purpose, the emitting means 8 and/or the detecting means 9 are distanced from the lying plane 7 and between them and the lying plane 7 form an operating zone 10 where, in use, a wooden plank 1 can be inserted. Moreover, in general, the emitting means 8 and the detecting means 9 are facing one another so as to define at least a first propagation plane in which one or more first directions of propagation, which can be parallel or diverging to each other, lie, and a second propagation plane (hereinafter also referred to simply as the first plane $P\alpha$ and the second plane $P\beta$) in which one or more second directions of propagation, which can be parallel or diverging to each other, lie; moreover the first plane $P\alpha$ and the second plane $P\beta$ for the propagation of the microwave beams from the emitting means 8 to the detecting means 9, are separate and far enough apart. Both the first plane $P\alpha$ and the second plane $P\beta$ pass through the operating zone 10. In most of the embodiments this result may be achieved mainly by using one of the following possible alternatives (even if hybrid solutions are also possible).

The first possibility is that the emitting means 8 comprise a microwave emitter for each propagation plane $P\alpha$, $P\beta$ and that the detecting means 9 also comprise a microwave detector for each propagation plane $P\alpha$, $P\beta$.

The second, preferred, possibility is that the emitting means 8 comprise a single microwave emitter for all of the propagation planes $P\alpha$, $P\beta$ (which can therefore emit microwaves in all of the propagation planes $P\alpha$, $P\beta$ to be used) and in contrast the detecting means 9 comprise a microwave detector for each propagation plane.

Finally, the third possibility is that the detecting means 9 comprise only a single microwave detector for all of the propagation planes $P\alpha$, $P\beta$ (which can therefore receive from several directions) and the emitting means 8 comprise a separate microwave emitter for each propagation plane.

To guarantee reliable results, in general the first plane $P\alpha$ forms a first minimum angle $\alpha1$ with the lying plane 7 (that is to say, the smallest angle of the two $\alpha1$,

α2 formed by the first plane Pα with the lying plane 7) which is not less than 30° and which intersects the lying plane 7 along a first line which is transversal, but preferably orthogonal, to the longitudinal axis (in Figure 7 it is orthogonal and is therefore perpendicular to the sheet and to plane XZ). Similarly, the second plane Pβ forms a second minimum angle β1 with the lying plane 7 (that is to say, the smallest angle of the two β1, β2 formed by the second plane Pβ with the lying plane 7) which is not less than 30° and which intersects the lying plane 7 along a second transversal line which is parallel with the first transversal line. Advantageously, in the preferred embodiments, the first minimum angle α1 and the second minimum angle β1 are between 30° and 60°. Finally, the first plane Pα and the second plane Pβ between them form a third minimum angle (αβ) which is not less than 30°.

Depending on requirements, the emitting means 8 and the detecting means 9 may either define only two propagation planes Pα, Pβ, or they may define three or more of them. In the former case, it is possible that both the first plane Pα and the second plane Pβ are angled on opposite sides relative to the lying plane 7, advantageously forming the same minimum angle α1, β1 with it (symmetrical solution similar to that of Figure 7 although in that Figure there is also a third plane Pγ), or that the first plane Pα is orthogonal to the lying plane 7 and that the second minimum angle β1 is between 30° and 60°. However, the preferred embodiment of this invention is of the latter type and is schematically illustrated in Figure 7. In this case, the emitting means 8 and the detecting means 9 are facing one another so as to also define a third propagation plane (in the following third plane Pγ) of the microwave beams from the emitting means 8 to the detecting means 9, the third plane Pγ being separate from the first plane Pα and the second plane Pβ and passing through the operating zone 10. The first and second planes Pα, Pβ are angled on opposite sides relative to the lying plane 7, and they form the same minimum angle α1, β1 with it and are symmetrical relative to the third plane Pγ. In fact, the latter is orthogonal to the lying plane 7 and intersects the lying plane 7 along a third line which is transversal to the nominal direction of the fibres N and also parallel with the first transversal line (therefore also perpendicular to the sheet in Figure 7 and to plane XZ). In this case the third plane Pγ forms both with the first plane Pα and with the second plane Pβ a fourth minimum angle αγ, γβ which is not less than 30°. Consequently, the first plane Pα and the second plane Pβ between them form a third minimum angle αβ of at least 60°.

Finally, in the preferred embodiment, to allow an analysis of the entire plank 1, portion after portion, the device 5 also comprises translation means 11 for in use translating the plank 1 relative to the emitting means 8 and to the detecting means 9 or vice versa. In the case illustrated in Figure 7 the translation means 11 allow the plank 1 to be shifted parallel with the longitudinal axis and could consist of one or more conveyors (advantageously of the belt or chain type).

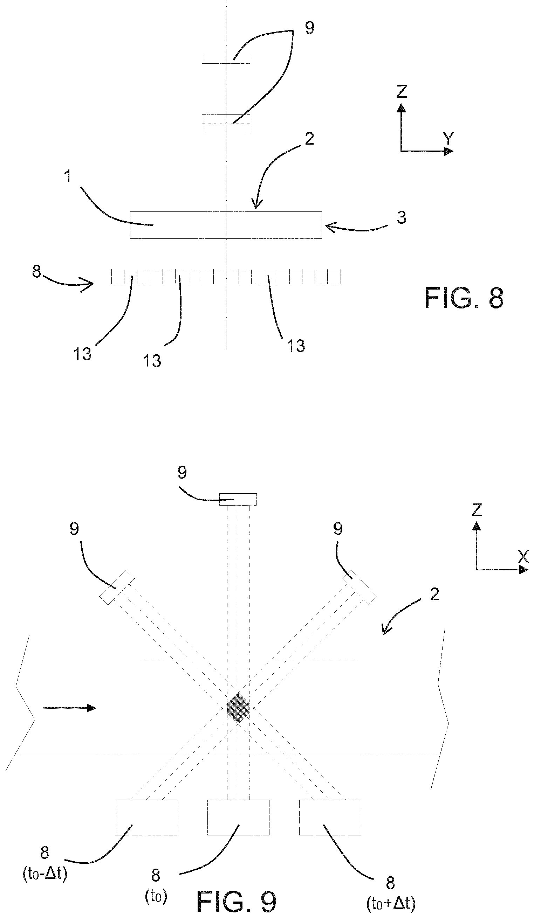

In the embodiment illustrated in Figures 7 and 8, in particular the emitting means 8 comprise a single emitter, whilst the detecting means 9 comprise three detectors mounted on a single supporting structure 12. The central detector is aligned with the emitter according to a propagation plane (the third plane Pγ) orthogonal to the lying plane 7 (and therefore in use to the first face 2), whilst the two lateral detectors are specular relative to one another and are aligned with the emitter according to propagation planes Pα, Pβ which form a 45° angle with the propagation plane Pγ of the central detector.

Depending on the embodiments, it is possible that each emitter comprises either a single emitting antenna 13 as in the embodiment illustrated in Figure 7 (which advantageously covers the entire width of the plank 1) or two or more emitting antennas 13 which are aligned (as shown in Figure 8). However, in both cases the emitter advantageously extends parallel with the first transversal line.

In the same way, each receiver may also comprise one or more receiving antennas which are aligned in a direction parallel with the first transversal line. However, in the accompanying drawings each receiver comprises only a single receiving antenna whose width is less than that of the plank 1. Returning to the apparatus 4 as a whole, in it the device 5 describe so far is mounted in such a way that the lying plane 7 is orthogonal to the reference plane XZ and in such a way that the longitudinal axis is, in contrast, parallel with it.

The apparatus 4 also comprises at least one electronic control and processing unit 14 operatively connected at least to the emitting means 8 and to the detecting means 9 and programmed to estimate the projection of the orientation of the fibres on the reference plane XZ by processing what was detected by the detecting means 9, and in particular by processing the amplitude and phase of the microwaves received.

However, in the preferred embodiment the electronic control and processing unit 14 is programmed to implement at least the steps of emitting, detecting, determining and estimating of the method according to this invention and described below.

Therefore what follows is a description of the method according to this invention for estimating the projection on a reference plane XZ of the direction of extension of the fibres L of a portion of a wooden plank 1. It shall be understood that what has already been described with reference to the apparatus 4 is also valid for corresponding elements of the method (that is to say, elements identified with the same definitions).

The method comprises first an operating step of identifying a portion of the plank 1 for which the orientation of the projection is to be estimated (as indicated below, the estimate of the projection for the entire plank 1 can be carried out as a sequence of estimates for individual portions), and an operating step of placing the plank 1 with its first face 2 orthogonal to the reference plane XZ and with the main axis of extension parallel with the reference plane XZ. If the apparatus 4 described above is being used, that is achieved by placing the first face 2, or the face opposite to and parallel with it, on the lying plane 7.

At this point the method comprises a first operating step of emitting at least a first polarized microwave beam (advantageously with plane/linear polarization in one plane) towards the portion of the plank 1 being examined, according to a first direction of incidence which lies in a first propagation plane $P\alpha$. As already indicated, the first plane $P\alpha$ will form a first minimum angle $\alpha 1$ with the first face 2 which is not less than 30° and will intersect the first face 2 along the first transversal line.

Simultaneously or at a different time, the method comprises a second operating step of emitting at least a second polarized microwave beam (advantageously with polarization similar to that of the first beam) towards the portion of the plank 1 being examined, according to a second direction of incidence which lies in a second propagation plane $P\beta$. As already indicated, the second plane $P\beta$ will form a second minimum angle $\beta 1$ with the first face 2 which is not less than 30° and will intersect the first face 2 along the second transversal line. Moreover, the second plane $P\beta$ will form with the first plane $P\alpha$ a third minimum angle $\alpha\beta$ which is not less than 30°.

Reference should be made to what is described above regarding the possible orientations of the first plane $P\alpha$ and the second plane $P\beta$.

Substantially simultaneously respectively with the first and second emitting steps (except for the delay due to the time taken by the microwaves to travel from the emitter to the receiver), the method comprises a first operating step of detecting, on the opposite side of the wood to that where the microwaves entered, the residual part of the first beam which passed through the plank 1, and a second operating step of detecting, again on the opposite side of the wood to that where the microwaves entered, the residual part of the second beam which passed through the plank 1.

At that point, the method comprises a first operating step of determining the amplitude and the phase of the residual part of the first beam which passed through the plank 1 (hereinafter indicated as the first amplitude and the first phase) and a second operating step of determining the amplitude and the phase of the residual part of the second beam which passed through the plank 1 (hereinafter indicated as the second amplitude and the second phase).

Finally, the method comprises an operating step of estimating the orientation of the projection of the direction of the fibres L on the reference plane XZ, by processing the first and second amplitudes and the first and second phases previously determined. That processing is effectively used to determine the projection on the reference plane XZ of the direction of the smallest semi-axis of the ellipsoid characteristic of the dielectric tensor of the wood.

However, in the preferred embodiment the method also comprises a third operating step of emitting at least a third polarized microwave beam (in a similar way to the first two) towards the portion of plank 1 being examined, according to a third direction of incidence which lies in a third plane $P\gamma$ orthogonal to the first face 2 and which intersects the first face 2 along the third transversal line. As already indicated, the third plane $P\gamma$ will form both with the first plane $P\alpha$ and with the second plane $P\beta$ a fourth minimum angle $\alpha\gamma$, $\gamma\beta$ which is not less than 30°, advantageously equal with both.

Consequently, the method comprises a third operating step of detecting, on the opposite side to the first face 2, the residual part of the third beam which passed through the plank 1, and a third operating step of determining the amplitude and the phase of that residual part detected (hereinafter indicated as the third amplitude and the third phase).

In this more complete embodiment of the method, the step of estimating the projection occurs by means of processing of the first, second and third amplitudes and of the first, second and third phases previously determined. The aim (implicit or explicit) is always to determine the smallest semi-axis of the ellipsoid.

Depending on the embodiments, it may be appropriate that the steps of emitting, and detecting of each beam occur either while keeping the plank 1 stationary relative to the various beams (which may be generated either simultaneously or one after another) or translating the plank 1 after each beam has been emitted (therefore, the various beams will be emitted one after another).

That is due to the need to allow the beams to pass through the same fibres of the wood as far as possible. In fact, as is easy to infer, since the propagation planes are angled relative to each other, it is not possible to make each beam pass through exactly the same fibres of wood and only those fibres.

Figure 9 illustrates that circumstance with reference to three beams lying in planes $P\alpha$, $P\beta$, $P\gamma$ angled at 45° to one another which intersect at the centre of the plank 1. As can be seen, only the hatched portion of wood is common to the three beams. Other portions are only common to two beams, whilst still others only have one beam passing through them. The situation schematically illustrated in Figure 9 may be obtained both by using three separate emitters and three separate receivers which are suitably positioned, or with a single emitter (or detector) and three receivers (or respectively, emitters) which are arranged as shown in Figure 7. In former case Figure 7 effectively shows the structure of the apparatus 4 which can be used to implement the method, whilst in the latter case Figure 9 shows the superposing in a single view of three different moments which correspond to three different plank 1 positions. In particular, considering the continuous line rectangle below the plank 1 to be a single emitter, and the rectangles above the plank 1 to be receivers, the continuous line upper rectangle represents the receiver towards which the third beam propagates at the moment to. The dashed line receiver to the left of the continuous line receiver represents the receiver towards which the first beam propagates at the moment $t_0$-$\Delta t$, whilst the dashed line receiver to the right represents the receiver towards which the first beam propagates at the moment $t_0$+$\Delta t$.

The assessments would be the same considering a situation with a single receiver below the plank 1 and three emitters above it.

Therefore, in the application of the method according to this invention the first, second and third amplitudes and phases must always be assessed with reference to the same portion of plank 1 (or rather, with reference to microwave beams which pass through not just other wood but also a common portion - the portion hatched in Figure 9).

However, in the preferred embodiment the steps of emitting, detecting and determining relating to each beam are carried out for a plurality of separate portions of the plank 1. That is advantageously achieved by repeating the various steps by making the plank 1 translate longitudinally, at least in a relative fashion, and repeating the various steps at preset time intervals.

In general, if each emitter comprises two or more emitting antennas 13, it is also possible that:

- each emitting step involves the successive emission of a plurality of microwave sub-beams;
- each emitting step involves the emitting one after another of each sub-beam towards the portion of the wooden plank 1 or a part of it;
- each detecting step involves the detection one after another of the residual part of each sub-beam which passed through the plank 1; and
- each determining step involves determining the amplitude and the phase corresponding to those of the residual part of each sub-beam which passed through the plank 1. Advantageously, each sub-beam is directed towards a part of the portion of plank 1 which is at least partly separate from that towards which the other sub-beams of the same beam are directed (the portion of plank 1 is basically examined at a plurality of slices which are side by side and aligned parallel with the transversal lines). Finally, in this embodiment the step of estimating the projection is carried out by means of processing of the amplitudes and phases determined in this way.

Finally, the following is a description of a preferred embodiment of the method according to this invention, which may be implemented using an apparatus 4 similar to that illustrated in Figure 7 or 8.

The configuration selected therefore requires the use of three receivers, each consisting of a single receiving antenna, and the use of a single emitter which may comprise one or more emitting antennas 13 which are aligned in a row. The emitter is located below the plank 1, the receivers above and the propagation planes of the various beams are one perpendicular to the first face 2, and the other two specular and angled on opposite sides relative to the central one. Of course, in different embodiments, receivers can be placed below the plank 1 with the emitter above, as well as only one receiver and three emitters can be used.

T(r,t) is the overall number which represents the amplitude and the phase of the signal received by each receiver "r", transmitted by each emitting antenna 13 "t", where:

- the value of "r" may be 1 (first angled receiver), 2 (vertical receiver) or 3 (second angled receiver); and
- the value of "t" is from 1 to n, where n is the total number of emitting antennas 13 in the row;

Both the emitting antennas 13 and the receivers are also polarized in a plane perpendicular to the first face 2 and parallel with the nominal direction of the fibres N.

At that point, for each emitting antenna 13 (k=1..n), the angle $\omega$ formed by the projection of the direction of the fibres L on the reference plane XZ, relative to the nominal direction N, is:

$$\omega = 0.5 \operatorname{atan}\left(\frac{1}{\tan(\varphi)} \frac{E_{r(k)} - E_{l(k)}}{E_{r(k)} + E_{l(k)}}\right)$$

where

$$E_{l(k)} = real\left(\frac{T(1,k)}{T(2,k)}\right)$$

$$E_{r(k)} = real\left(\frac{T(3,k)}{T(2,k)}\right)$$

and $\varphi$ represents the absolute value of the angle formed inside the wood due to refraction, by the first beam and by the second beam relative to a direction normal to the first face 2 (as shown in Figure 6). The angle $\varphi$, which is determined by refraction phenomena, depends both on the type of wood, its density and its degree of moisture, and on the angle of incidence formed by the first plane P$\alpha$ and by the second plane P$\beta$ with the first face 2 of the plank 1.

This invention brings important advantages.

In fact, the method, the apparatus and the device according to this invention allow estimation of the projection on a reference plane of the direction of extension of the fibres of a portion of a wooden plank, examining a face of the plank which is orthogonal to the reference plane, and in particular they allow the determination of the dive angle relative to a main face of the plank by emitting the microwaves from above against said main face.

Finally, it should be noticed that this invention is relatively easy to produce and that even the cost linked to implementing the invention is not very high. The invention described above may be modified and adapted in several ways without thereby departing from the scope of the inventive concept. Moreover, all details of the invention may be substituted with other technically equivalent elements and the materials used, as well as the shapes and dimensions of the various components, may vary according to requirements.

**Claims**

1. A method for estimating the projection on a reference plane (XZ) of the direction of extension of the fibres (L) of a portion of a wooden plank (1), the wooden plank (1) having a main axis of extension, a first flat face (2) which is parallel with the main axis of extension and a nominal direction of the fibres (N) which is defined as parallel with the main axis of extension, the fibres having an orientation in space with any deviation relative to the nominal direction, the method comprising:

   an operating step of identifying a portion of the plank (1) for which the orientation of said projection is to be estimated;
   an operating step of placing the plank (1) with its first face (2) orthogonal to the reference plane (XZ) and with the main axis of extension parallel with the reference plane (XZ);
   the method further comprising:

   an operating step of emitting at least a first polarized microwave beam towards said portion of the plank (1) according to a first direction of incidence lying in a first plane (Pα) which forms a first minimum angle (α1) with said first face (2) which is not less than 30° and which intersects the first face (2) along a first line transversal to the nominal direction of the fibres (N), the first polarized microwave beam being emitted in such a way to enter the plank (1) through the first face (2); said first polarized microwave beam being polarized exclusively in a plane parallel with the reference plane (XZ);
   an operating step of emitting at least a second polarized microwave beam towards said portion of the plank (1) according to a second direction of incidence lying in a second plane (Pβ) which forms a second minimum angle (β1) with said first face (2) which is not less than 30° and which intersects the first face (2) along a second line transversal to the nominal direction of the fibres (N) and parallel with the first transversal line, the second polarized microwave beam being emitted in such a way to enter the plank (1) through the first face (2); the second plane (Pβ) also forming with the first plane (Pα) a third minimum angle (αβ) which is not less than 30°; said second polarized microwave beam being polarized exclusively in a plane parallel with the reference plane (XZ);
   an operating step of detecting the residual part of the first beam which passed through the plank (1);
   an operating step of detecting the residual part of the second beam which passed through the plank (1);
   an operating step of determining a first amplitude and a first phase corresponding to those of the residual part of the first beam which passed through the plank (1);
   an operating step of determining a second amplitude and a second phase corresponding to those of the residual part of the second beam which passed through the plank (1); and
   an operating step of processing the first and second amplitudes and the first and second phases previously determined, to estimate the orientation of the projection of the direction of extension of the fibres (L) on the reference plane (XZ) which is orthogonal to the first face (2).

2. The method according to claim 1, **characterised:**

   **in that** the first plane (Pα) and the second plane (Pβ) are selected in such a way that they form the first minimum angle (α1) and the second minimum angle (β1) respectively on opposite sides with respect to a plane perpendicular to the nominal direction of the fibres (N); and/or
   **in that** the first plane (Pα) and the second plane (Pβ) are selected in such a way that the first minimum angle (α1) is equal to the second minimum angle (β1); and/or
   **in that** the first minimum angle (α1) and the second minimum angle (β1) are selected so that they are between 30° and 60°; or
   **in that** the first plane (Pα) is selected orthogonal to the first face (2), and in that the second minimum angle (β1) is selected so that it is between 30° and 60°.

3. The method according to claim 1, **characterised in that** the first plane (Pα) and the second plane (Pβ) are selected in such a way that the first minimum angle (α1) is equal to the second minimum angle (β1), and **in that** it also comprises:

an operating step of emitting at least a third polarized microwave beam towards said portion of the plank (1) according to a third direction of incidence lying in a third plane (Pγ) which is orthogonal to the first face (2) and which intersects the first face (2) along a third line transversal to the nominal direction of the fibres (N) and parallel with the first transversal line; the third plane (Pγ) forming with both the first plane (Pα) and the second plane (Pβ) a fourth minimum angle (αγ, γβ) which is not less than 30°;

an operating step of detecting the residual part of the third beam which passed through the plank (1); and

an operating step of determining a third amplitude and a third phase corresponding to those of the residual part of the third beam which passed through the plank (1);

and **in that** the third amplitude and the third phase previously determined are processed together with the first and second amplitudes and the first and second phases, in order to estimate the orientation of said projection.

4. The method according to claims 3 , **characterised in that** the third beam has the same polarization as the first and second beams.

5. The method according to any one of the preceding claims, **characterised in that** the first transversal line is selected orthogonal to the nominal direction of the fibres (N).

6. The method according to any one of the preceding claims, **characterised in that**:

each emitting step involves the emitting one after another of a plurality of microwave sub-beams towards a part of the portion of plank (1) which is at least partly shifted along a direction perpendicular to the reference plane (XZ) with respect to that towards which the other sub-beams of the same beam are directed;

each detecting step involves the detection one after another of the residual part of each sub-beam which passed through the plank (1);

each determining step involves determining the amplitude and the phase corresponding to those of the residual part of each sub-beam which passed through the plank (1); and

the amplitudes and phases determined in this way are processed in order to estimate the orientation of said projection.

7. A device for examining the direction of extension of the fibres (L) of a wooden plank (1), comprising:

supporting means (6) for the plank (1) which define both a lying plane (7) and a longitudinal axis, the supporting means being suitable to receive the plank (1) in such a way that, during operation, the plank (1) lies with a first flat face (2) parallel with the lying plane (7) and a nominal direction of the fibres (N), which is defined as parallel with a main axis of extension of the plank (1) that is also parallel with the first flat face (2), is parallel with the longitudinal axis;

means for emitting (8) polarized microwave beams which are positioned on a first side of the lying plane (7); said polarized microwave beams being polarized exclusively in a plane parallel with a reference plane (XZ) which is orthogonal to the lying plane (7) and parallel with the longitudinal axis;

means for detecting (9) microwaves which are positioned on a second side of the lying plane (7) opposite to the first side, for in use detecting the residual part of the polarized microwave beams emitted by the emitting means (8) after they have passed through the plank (1);

the emitting means (8) and/or the detecting means (9) being distanced from the lying plane (7) and between them and the lying plane (7) forming an operating zone (10) where, in use, the wooden plank (1) can be inserted;

the emitting means (8) and the detecting means (9) facing one another so as to define at least a first plane (Pα) and a second plane (Pβ) which are separate for the propagation of the microwave beams from the emitting means (8) to the detecting means (9), both of said planes passing through the operating zone (10);

wherein:

the first plane (Pα) intersects the lying plane (7) along a first line which is transversal to the longitudinal axis, forming a first minimum angle (α1) with the lying plane (7) which is not less than 30°; and

the second plane (Pβ) intersects the lying plane (7) along a second transversal line which is transversal to the longitudinal axis and parallel with the first transversal line, forming a second minimum angle (β1) with the lying plane (7) which is not less than 30°, the second plane (Pβ) also forming with the first plane (Pα) a third minimum angle (αβ) which is not less than 30°.

8. The device according to claim 7, **characterised:**

**in that** the first plane (Pα) and the second plane (Pβ) form the first minimum angle (α1) and the second minimum angle (β1) respectively on opposite sides with respect to a plane perpendicular to the nominal direction of the fibres (N); and/or

**in that** the first minimum angle ($\alpha$1) is equal to the second minimum angle ($\beta$1); and/or

**in that** the first minimum angle ($\alpha$1) and the second minimum angle ($\beta$1) are between 30° and 60°; or in that the first plane (P$\alpha$) is orthogonal to the lying plane (7), and in that the second minimum angle ($\beta$1) is between 30° and 60°.

9. The device according to claim 8, **characterised in that** the first minimum angle ($\alpha$1) is equal to the second minimum angle ($\beta$1) and **in that** the emitting means (8) and the detecting means (9) are facing one another so as to also define at least a third plane (P$\gamma$) for propagation of the microwave beams from the emitting means (8) to the detecting means (9), separate from the first plane (P$\alpha$) and from the second plane (P$\beta$) and passing through the operating zone (10); the third plane (P$\gamma$) being orthogonal to the lying plane (7) and intersecting the lying plane (7) along a third line transversal to the nominal direction of the fibres (N) and parallel with the first transversal line; the third plane (P$\gamma$) forming both with the first plane (P$\alpha$) and with the second plane (P$\beta$) a fourth minimum angle which is not less than 30°.

10. The device according to any one of the claims from 7 to 9, **characterised in that** the first transversal line is orthogonal to the longitudinal axis.

11. The device according to any one of the claims from 7 to 10, **characterised in that** alternatively:

    - the emitting means (8) comprise a microwave emitter for each propagation plane and the detecting means (9) comprise a microwave detector for each propagation plane; or
    - the emitting means (8) comprise a single microwave emitter for all of the propagation planes and the detecting means (9) comprise a microwave detector for each propagation plane; or
    - the detecting means (9) comprise a single microwave detector for all of the propagation planes and the emitting means (8) comprise a microwave emitter for each propagation plane.

12. The device according to claim 11, **characterised in that** each emitter comprises one or more emitting antennas (13) which are aligned in a direction parallel with the first transversal line, and/or **in that** each emitter comprises one or more emitting antennas (13) which are aligned in a direction parallel with the first transversal line and each receiver comprises one or more receiving antennas which are aligned in a direction parallel with the first transversal line.

13. An apparatus for estimating the projection on a reference plane (XZ) of the direction of extension of the fibres (L) of a portion of a wooden plank (1), the wooden plank (1) having a nominal direction of the fibres (N) which is parallel with a main axis of extension of it and a first face (2) which is also parallel with the main axis of extension;

the apparatus (4) comprising:

    at least one device (5) made according to any one of the claims from 7 to 12,; and
    at least one electronic control and processing unit (14) operatively connected at least to the emitting means (8) and to the detecting means (9) and programmed to process what was detected by the detecting means (9) in order to estimate said projection.

14. The apparatus according to claim 13, **characterised in that** the electronic control and processing unit (14) is programmed to implement at least the steps of emitting, detecting, determining and processing of the method according to any one of the claims from 1 to 6.

**Patentansprüche**

1. Ein Verfahren zur Schätzung der Projektion auf einer Referenzebene (XZ) der Ausdehnungsrichtung der Fasern (L) eines Teils eines Holzbretts (1), wobei das Holzbrett (1) eine Hauptausdehnungsachse hat, eine erste flache Seite (2), die parallel zur Hauptausdehnungsachse ist, und eine Nennrichtung der Fasern (N), die als parallel zur Hauptausdehnungsachse definiert ist, wobei die Fasern eine Ausrichtung im Raum mit einer Abweichung in Bezug zur Nennrichtung haben, dabei umfasst das Verfahren:

    einen Arbeitsschritt des Identifizierens eines Teils des Bretts (1), für den die Ausrichtung besagter Projektion zu schätzen ist;
    einen Arbeitsschritt des Platzierens des Bretts (1) mit seiner ersten Seite (2) im rechten Winkel zur Referenzebene (XZ) und mit der Hauptausdehnungsachse parallel zur Referenzebene (XZ);
    wobei das Verfahren ferner umfasst:

    einen Arbeitsschritt des Sendens mindestens eines ersten polarisierten Mikrowellenstrahls zu besagtem Teil des Bretts (1), übereinstimmend mit einer ersten Einfallsrichtung, welche auf einer ersten Ebene (P$\alpha$) liegt, welche einen ersten Mindestwinkel ($\alpha$1) zusammen mit besagter erster Seite (2) bildet, welcher nicht weniger als 30° beträgt und welcher die erste Seite (2) auf einer ersten Linie quer zur Nennrichtung der Fasern (N) schneidet, wobei der erste polarisierte Mikrowellenstrahl solcherart gesendet wird, dass er in das Brett (1) durch die erste Seite (2) eintritt; wobei besagter erster polarisierter Mikrowellenstrahl ausschließlich auf einer Ebene parallel zur Referenzebene (XZ) polarisiert wird;

einen Arbeitsschritt des Sendens mindestens eines zweiten polarisierten Mikrowellenstrahls zu besagtem Teil des Bretts (1) in Übereinstimmung mit einer zweiten Einfallsrichtung, welche auf einer zweiten Ebene (Pβ) liegt, welche einen zweiten Mindestwinkel (β1) mit besagter erster Seite (2) bildet, welcher nicht weniger als 30° beträgt und welcher die erste Seite (2) auf einer zweiten Linie quer zur Nennrichtung der Fasern (N) und parallel zur ersten Querlinie schneidet, wobei der zweite polarisierte Mikrowellenstrahl solcherart gesendet wird, dass er in das Brett (1) durch die erste Seite (2) eintritt; die zweite Ebene (Pβ) bildet dabei zusammen mit der ersten Ebene (Pα) einen dritten Mindestwinkel (αβ), der nicht weniger als 30° beträgt; besagter zweiter polarisierter Mikrowellenstrahl wird dabei ausschließlich auf einer Ebene polarisiert, die parallel zur Referenzebene (XZ) liegt;

einen Arbeitsschritt des Erfassens des restlichen Teils des erstens Strahls, der durch das Brett (1) drang;

einen Arbeitsschritt des Erfassens des restlichen Teils des zweiten Strahls, der durch das Brett (1) drang;

einen Arbeitsschritt der Bestimmung einer ersten Amplitude und einer ersten Phase, welche denjenigen des restlichen Teils des ersten Strahls, der durch das Brett (1) drang, entsprechen; und

einen Arbeitsschritt der Bestimmung einer zweiten Amplitude und einer zweiten Phase, welche denjenigen des restlichen Teils des zweiten Strahls, der durch das Brett (1) drang, entsprechen; und

einen Arbeitsschritt der Verarbeitung der ersten und der zweiten Amplitude sowie der ersten und der zweiten Phase, die vorher bestimmt wurden, um die Ausrichtung der Projektion der Ausdehnungsrichtung der Fasern (L) auf der Referenzebene (XZ), welche im rechten Winkel zur ersten Seite (2) ist, zu schätzen.

2. Das Verfahren nach Patentanspruch 1, **gekennzeichnet dadurch:**

> **dass** die erste Ebene (Pα) und die zweite Ebene (Pβ) solcherart gewählt werden, dass sie den ersten Mindestwinkel (α1) und den zweiten Mindestwinkel (β1) jeweils an gegenüberliegenden Seiten in Bezug auf eine Ebene lotrecht zur Nennrichtung der Fasern (N) bilden; und/oder
**dass** die erste Ebene (Pα) und die zweite Ebene (Pβ) solcherart gewählt werden, dass der erste Mindestwinkel (α1) gleich dem zweiten Mindestwinkel (β1) ist; und/oder
**dass** der erste Mindestwinkel (α1) und der zweite Mindestwinkel (β1) so gewählt werden, dass sie zwischen 30° und 60° betragen; oder
**dass** die erste Ebene (Pα) im rechten Winkel zur ersten Seite (2) gewählt wird und dass der zweite Mindestwinkel (β1) so gewählt wird, dass er zwischen 30° und 60° beträgt.

3. Das Verfahren nach Patentanspruch 1, **gekennzeichnet dadurch, dass** die erste Ebene (Pα) und die zweite Ebene (Pβ) solcherart gewählt werden, dass der erste Mindestwinkel (α1) gleich dem zweiten Mindestwinkel (β1) ist, und dadurch, dass es auch Folgendes umfasst:

> einen Arbeitsschritt des Sendens mindestens eines dritten polarisierten Mikrowellenstrahls zu besagtem Teil des Bretts (1) in Übereinstimmung mit einer dritten Einfallsrichtung auf einer dritten Ebene (Pγ), welche im rechten Winkel zur ersten Seite (2) ist und welcher die erste Seite (2) auf einer dritten Linie quer zur Nennrichtung der Fasern (N) und parallel zur ersten Querlinie schneidet, wobei die dritte Ebene (Pγ) zusammen sowohl mit der ersten Ebene (Pα) als auch mit der zweiten Ebene (Pβ) einen vierten Mindestwinkel (αγ, γβ) bildet, welcher nicht weniger als 30° beträgt,
> einen Arbeitsschritt des Erfassens des restlichen Teils des dritten Strahls, der durch das Brett (1) drang; und
> einen Arbeitsschritt der Bestimmung einer dritten Amplitude und einer dritten Phase, welche denjenigen des restlichen Teils des dritten Strahls, der durch das Brett (1) drang, entsprechen;
> und dadurch, dass die dritte Amplitude und die dritte Phase, die vorher bestimmt wurden, zusammen mit der ersten und der zweiten Amplitude sowie mit der ersten und der zweiten Phase verarbeitet werden, um die Ausrichtung besagter Projektion zu schätzen.

4. Das Verfahren nach Patentanspruch 3, **gekennzeichnet dadurch, dass** der dritte Strahl dieselbe Polarisierung wie der erste und der zweite Strahl aufweist.

5. Das Verfahren nach einem der vorherigen Patentansprüche, **gekennzeichnet dadurch, dass** die erste Querlinie im rechten Winkel zur Nennrichtung der Fasern (N) gewählt ist.

6. Das Verfahren nach einem der vorherigen Patentansprüche, **gekennzeichnet dadurch, dass**:

   jeder Schritt des Sendens das Senden einer Vielzahl von Mikrowellenunterstrahlen einen nach dem anderen in Richtung eines Brettteils (1) beinhaltet, welcher zumindest teilweise in eine Richtung lotrecht zur Referenzebene (XZ) in Bezug auf diejenige, in welche die anderen Unterstrahlen desselben Strahls gerichtet sind, verschoben wird;

   jeder Schritt des Erfassens die Erfassung des restlichen Teils jedes Unterstrahls, der durch das Brett (1) drang, einen nach dem anderen beinhaltet;

   jeder Schritt des Bestimmens die Bestimmung der Amplitude und der Phase beinhaltet, welche denjenigen des restlichen Teils jedes Unterstrahls, der durch das Brett (1) drang, entsprechen; und

   die Amplituden und Phasen, die solcherart bestimmt wurden, verarbeitet werden, um die Ausrichtung besagter Projektion zu schätzen.

7. Eine Vorrichtung zur Prüfung der Ausdehnungsrichtung der Fasern (L) eines Holzbretts (1), die Folgendes umfasst:

   Haltemittel (6) für das Brett (1), welche sowohl eine liegende Ebene (7) als auch eine Längsachse definieren, wobei die Haltemittel dazu geeignet sind, das Brett (1) solcherart zu umfassen, dass das Brett (1) während der Anwendung mit einer ersten, flachen Seite (2) parallel zur liegenden Ebene (7) liegt, und eine Nennrichtung der Fasern (N), welche als parallel zu einer Hauptausdehnungsachse des Bretts (1) definiert ist, welche auch parallel zur ersten flachen Seite (2) ist, ist parallel zur Längsachse;

   Mittel zum Senden (8) polarisierter Mikrowellenstrahlen, welche auf einer ersten Seite der liegenden Ebene (7) positioniert sind; besagte polarisierte Mikrowellenstrahlen werden dabei ausschließlich auf einer Ebene parallel zu einer Referenzebene (XZ) polarisiert, welche im rechten Winkel zur liegenden Ebene (7) und parallel zur Längsachse ist;

   Mittel zur Erfassung (9) von Mikrowellen, welche auf einer zweiten Seite der liegenden Ebene (7) gegenüber zur ersten Seite positioniert sind, um während der Anwendung den restlichen Teil der polarisierten Mikrowellenstrahlen zu erfassen, die durch die Sendemittel (8) gesendet werden, nachdem sie durch das Brett (1) gedrungen sind;

   die Sendemittel (8) und/oder die Erfassungsmittel (9) befinden sich dabei in einem Abstand von der liegenden Ebene (7) und zwischen sich und der liegenden Ebene (7) bilden sie einen Arbeitsbereich (10), in den das Holzbrett (1) während der Anwendung eingefügt werden kann; dabei sind die Sendemittel (8) und die Erfassungsmittel (9) zueinander gerichtet, sodass sie mindestens eine erste Ebene (Pα) und eine zweite Eben (Pβ), welche getrennt sind, zur Verbreitung der Mikrowellenstrahlen von den Sendemitteln (8) zu den Erfassungsmitteln (9) bilden, dabei führen beide besagten Ebenen durch den Arbeitsbereich (10);

   dabei:

   schneidet die erste Ebene (Pα) die liegende Ebene (7) auf einer ersten Linie, welche quer zur Längsachse ist, wobei sie einen ersten Mindestwinkel (α1) mit der liegenden Ebene (7) bildet, welcher nicht weniger als 30° beträgt; und die zweite Ebene (Pβ) schneidet die liegende Ebene (7) auf einer zweiten Querlinie, welche quer zur Längsachse und parallel zur ersten Querlinie ist und einen zweiten Mindestwinkel (β1) mit der liegenden Ebene (7) bildet, welcher nicht weniger als 30° beträgt, wobei die zweite Ebene (Pβ) auch zusammmen mit der ersten Ebene (Pα) einen dritten Mindestwinkel (αβ) bildet, welcher nicht weniger als 30° beträgt.

8. Die Vorrichtung nach Patentanspruch 7, **gekennzeichnet:**

   **dadurch, dass** die erste Ebene (Pα) und die zweite Ebene (Pβ) den ersten Mindestwinkel (α1) und den zweiten Mindestwinkel (β1) jeweils auf gegenüberliegenden Seiten in Bezug auf eine Ebene bilden, welche lotrecht zur Nennrichtung der Fasern (N) liegt; und/oder

   dadurch, dass der erste Mindestwinkel (α1) gleich dem zweiten Mindestwinkel (β1) ist; und/oder

   dadurch, dass der erste Mindestwinkel (α1) und der zweite Mindestwinkel (β1) zwischen 30° und 60° betragen; oder dadurch, dass die erste Ebene (Pα) im rechten Winkel zur liegenden Ebene (7) ist, und dadurch, dass der zweite Mindestwinkel (β1) zwischen 30° und 60° beträgt.

9. Die Vorrichtung nach Patentanspruch 8, **gekennzeichnet dadurch, dass** der erste Mindestwinkel (α1) gleich dem zweiten Mindestwinkel (β1) ist und dadurch, dass die Sendemittel (8) und die Erfassungsmittel (9) zueinander gerichtet sind, sodass sie auch mindestens eine dritte Ebene (Pγ) zur Verbreitung der Mikrowellenstrahlen von den Sendemitteln (8) zu den Erfassungsmitteln (9) definieren, welche getrennt von der ersten Ebene (Pα) und von der zweiten Ebene (Pβ) ist und durch den Arbeitsbereich (10) verläuft; wobei die dritte Ebene (Pγ) im rechten Winkel zur liegenden Ebene (7) ist und liegende Ebene (7) auf einer dritten Linie quer zur Nennrichtung der Fasern (N) und parallel zur ersten Querlinie schneidet; wobei die dritte Ebene (Pγ) dabei zusammen sowohl mit der ersten Ebene (Pα) als auch mit der zweiten Ebene (Pβ) einen vierten Mindestwinkel bildet, welcher nicht weniger als 30° beträgt.

10. Die Vorrichtung nach einem der Patentansprüche 7 bis 9, **gekennzeichnet dadurch, dass** die erste Querlinie im rechten Winkel zur Längsachse verläuft.

11. Die Vorrichtung nach einem der Patentansprüche 7 bis 10, **gekennzeichnet durch** folgende Alternativen:

> - dass die Sendemittel (8) einen Mikrowellensender für jede Verbreitungsebene umfassen und dass die Erfassungsmittel (9) einen Mikrowellendetektoren für jede Verbreitungsebene umfassen; oder
> - dass die Sendemittel (8) einen einzigen Mikrowellensender für alle Verbreitungsebenen umfassen und dass die Erfassungsmittel (9) einen Mikrowellendetektoren für jede Verbreitungsebene umfassen; oder
> - dass die Erfassungsmittel (9) einen einzigen Mikrowellendetektoren für alle Verbreitungsebenen umfassen und dass die Sendemittel (8) einen Mikrowellensender für jede Verbreitungsebene umfassen.

12. Die Vorrichtung nach Patentanspruch 11, **gekennzeichnet dadurch, dass** jeder Sender eine oder mehrere Sendeantennen (13) umfasst, welche in einer Richtung parallel zur ersten Querlinie ausgerichtet sind, und/oder dadurch, dass jeder Sender eine oder mehrere Sendeantennen (13) umfasst, welche in einer Richtung parallel zur ersten Querlinie ausgerichtet sind und dass jeder Empfänger eine oder mehrere Empfangsantennen umfasst, welche in einer Richtung parallel zur ersten Querlinie ausgerichtet sind.

13. Ein Apparat zur Schätzung der Projektion auf einer Referenzebene (XZ) der Ausdehnungsrichtung der Fasern (L) eines Teils eines Holzbretts (1), wobei das Holzbrett (1) eine Nennrichtung der Fasern (N) hat, welche parallel zu einer Hauptausdehnungsachse davon ist, und eine erste Seite (2) hat, welche ebenfalls parallel zur Hauptausdehnungsachse ist; dabei umfasst der Apparat (4):

mindestens eine Vorrichtung (5), die in Übereinstimmung mit einem der Patentansprüche von 7 bis 12 gefertigt ist; und
mindestens eine elektronische Steuerungs- und Verarbeitungseinheit (14), welche operativ mindestens mit den Sendemitteln (8) und den Erfassungsmittel (9) verbunden ist und programmiert ist, um zu verarbeiten, was durch die Erfassungsmittel (9) erfasst wurde, um besagte Projektion zu schätzen.

14. Der Apparat nach Patentanspruch 13, **gekennzeichnet dadurch, dass** die elektronische Steuerungs- und Verarbeitungseinheit (14) programmiert ist, um mindestens die Schritte des Sendens, Erfassens, Bestimmens und Verarbeitens des Verfahrens nach einem der Patentansprüche von 1 bis 6 auszuführen.

## Revendications

1. Un procédé pour estimer la projection sur un plan de référence (XZ) de la direction d'extension des fibres (L) d'une portion d'une planche de bois (1), la planche de bois (1) ayant un axe d'extension principal, une première face plate (2) qui est parallèle à l'axe d'extension principal et une direction nominale des fibres (N) qui est définie comme étant parallèle à l'axe d'extension principal, les fibres ayant une orientation dans l'espace avec n'importe quel écart par rapport à la direction nominale, le procédé comprenant :

> une phase opérationnelle consistant à identifier une portion de la planche (1) pour laquelle l'orientation de ladite projection doit être estimée ;
> une phase opérationnelle consistant à placer la planche (1) avec sa première face (2) orthogonale au plan de référence (XZ) et avec l'axe d'extension principal parallèle au plan de référence (XZ) ;
> le procédé comprenant en outre :

>> une phase opérationnelle consistant à émettre au moins un premier faisceau de micro-ondes polarisé vers ladite portion de la planche (1) selon une première direction d'incidence située dans un premier plan (Pα) qui forme un premier angle minimum (α1) avec ladite première face (2) qui n'est pas inférieur à 30° et qui coupe la première face (2) le long d'une première ligne transversale à la direction nominale des fibres (N), le premier faisceau de micro-ondes polarisé étant émis de manière à entrer dans la planche (1) à travers la première face (2) ;

ledit premier faisceau de micro-ondes polarisé étant polarisé exclusivement dans un plan parallèle au plan de référence (XZ) ;

une phase opérationnelle consistant à émettre au moins un deuxième faisceau de micro-ondes polarisé vers ladite portion de la planche (1) selon une deuxième direction d'incidence située dans un deuxième plan (Pβ) qui forme un deuxième angle minimum (β1) avec ladite première face (2) qui n'est pas inférieur à 30° et qui coupe la première face (2) le long d'une deuxième ligne transversale à la direction nominale des fibres (N) et parallèle à la première ligne transversale, le deuxième faisceau de micro-ondes polarisé étant émis de manière à entrer dans la planche (1) à travers la première face (2) ; le deuxième plan (Pβ) formant aussi avec le premier plan (Pα) un troisième angle minimum (αβ) qui n'est pas inférieur à 30° ; ledit deuxième faisceau de micro-ondes polarisé étant polarisé exclusivement dans un plan parallèle au plan de référence (XZ) ;

une phase opérationnelle consistant à détecter la partie résiduelle du premier faisceau qui a traversé la planche (1) ;

une phase opérationnelle consistant à détecter la partie résiduelle du deuxième faisceau qui a traversé la planche (1) ;

une phase opérationnelle consistant à déterminer une première amplitude et une première phase correspondant à celles de la partie résiduelle du premier faisceau qui a traversé la planche (1) ;

une phase opérationnelle consistant à déterminer une deuxième amplitude et une deuxième phase correspondant à celles de la partie résiduelle du deuxième faisceau qui a traversé la planche (1) ; et

une phase opérationnelle consistant à traiter les première et deuxième amplitudes et les première et deuxième phases précédemment déterminées, pour estimer l'orientation de la projection de la direction d'extension des fibres (L) sur le plan de référence (XZ) qui est orthogonal à la première face (2).

2. Le procédé selon la revendication 1, **caractérisé :**

en ce que le premier plan (Pα) et le deuxième plan (Pβ) sont choisis de manière à ce qu'ils forment le premier angle minimum (α1) et le deuxième angle minimum (β1) respectivement sur des côtés opposés par rapport à un plan perpendiculaire à la direction nominale des fibres (N) ; et/ou

**en ce que** le premier plan (Pα) et le deuxième plan (Pβ) sont choisis de manière à ce que le premier angle minimum (α1) soit égal au deuxième angle minimum (β1) ; et/ou

**en ce que** le premier angle minimum (α1) et le deuxième angle minimum (β1) sont choisis de manière à être compris entre 30° et 60° ; ou

**en ce que** le premier plan (Pα) est choisi orthogonal à la première face (2), et en ce que le deuxième angle minimum (β1) est choisi de manière à être compris entre 30° et 60°.

3. Le procédé selon la revendication 1, **caractérisé en ce que** le premier plan (Pα) et le deuxième plan (Pβ) sont choisis de manière à ce que le premier angle minimum (α1) soit égal au deuxième angle minimum (β1), et **en ce qu'**il comprend aussi :

une phase opérationnelle consistant à émettre au moins un troisième faisceau de micro-ondes polarisé vers ladite portion de la planche (1) selon une troisième direction d'incidence située dans un troisième plan (Pγ) qui est orthogonal à la première face (2) et qui coupe la première face (2) le long d'une troisième ligne transversale à la direction nominale des fibres (N) et parallèle à la première ligne transversale ; le troisième plan (Pγ) formant avec le premier plan (Pα) ainsi qu'avec le deuxième plan (Pβ) un quatrième angle minimum (αγ, γβ) qui n'est pas inférieur à 30° ;

une phase opérationnelle consistant à détecter la partie résiduelle du troisième faisceau qui a traversé la planche (1) ; et

une phase opérationnelle consistant à déterminer une troisième amplitude et une troisième phase correspondant à celles de la partie résiduelle du troisième faisceau qui a traversé la planche (1) ;

et **en ce que** la troisième amplitude et la troisième phase précédemment déterminées sont traitées avec les première et deuxième amplitudes et les première et deuxième phases, afin d'estimer l'orientation de ladite projection.

4. Le procédé selon la revendication 3, **caractérisé en ce que** le troisième faisceau a la même polarisation que les premier et deuxième faisceaux.

5. Le procédé selon l'une quelconque des revendications précédentes, **caractérisé en ce que** la première ligne transversale est choisie orthogonale à la direction nominale des fibres (N).

6. Le procédé selon l'une quelconque des revendications précédentes, **caractérisé en ce que** :

chaque phase d'émission prévoit l'émission, l'un après l'autre, d'une pluralité de sous-faisceaux de micro-ondes vers une partie de la portion de planche (1) qui est au moins partiellement décalée le long d'une direction perpendiculaire au plan de référence (XZ) par rapport à celles vers lesquelles sont dirigés les autres sous-faisceaux du même faisceau ;

chaque phase de détection prévoit la détection, l'une après l'autre, de la partie résiduelle de chaque sous-faisceau qui a traversé la planche (1) ;

chaque phase de détermination prévoit la détermination de l'amplitude et de la phase correspondant à celles de la partie résiduelle de chaque sous-faisceau qui a traversé la planche (1) ; et

les amplitudes et les phases ainsi déterminées sont traitées de manière à estimer l'orientation de ladite projection.

7. Un dispositif pour examiner la direction d'extension des fibres (L) d'une planche de bois (1), comprenant :

des moyens (6) de support de la planche (1) qui définissent à la fois un plan d'appui (7) et un axe longitudinal, les moyens de support étant destinés à recevoir la planche (1) de manière à ce que, en fonctionnement, la planche (1) repose avec une première face plate (2) parallèle au plan d'appui (7) et une direction nominale des fibres (N), qui est définie comme étant parallèle à un axe d'extension principal de la planche (1) qui est aussi parallèle à la première face plate (2), est parallèle à l'axe longitudinal ;

des moyens (8) d'émission de faisceaux de micro-ondes polarisés qui sont positionnés sur un premier côté du plan d'appui (7) ; lesdits faisceaux de micro-ondes polarisés étant polarisés exclusivement dans un plan parallèle à un plan de référence (XZ) qui est orthogonal au plan d'appui (7) et parallèle à l'axe longitudinal ;

des moyens (9) de détection des micro-ondes qui sont positionnés sur un deuxième côté du plan d'appui (7) opposé au premier côté pour détecter, pendant l'utilisation, la partie résiduelle des faisceaux de micro-ondes polarisés émis par les moyens d'émission (8) après leur passage à travers la planche (1) ;

les moyens d'émission (8) et/ou les moyens de détection (9) étant espacés du plan d'appui (7) et formant entre eux et le plan d'appui (7) une zone opérationnelle (10) où, pendant l'utilisation, la planche de bois (1) peut être insérée ;

les moyens d'émission (8) et les moyens de détection (9) étant les uns en face des autres de manière à définir au moins un premier plan (Pα) et un deuxième plan (Pβ) qui sont distincts pour la propagation des faisceaux de micro-ondes des moyens d'émission (8) aux moyens de détection (9), lesdits plans passant tous deux à travers la zone opérationnelle (10) ;

où :

le premier plan (Pα) coupe le plan d'appui (7) le long d'une première ligne qui est transversale à l'axe longitudinal, formant un premier angle minimum (α1) avec le plan d'appui (7) qui n'est pas inférieur à 30° ; et

le deuxième plan (Pβ) coupe le plan d'appui (7) le long d'une deuxième ligne transversale qui est transversale à l'axe longitudinal et parallèle à la première ligne transversale, formant un deuxième angle minimum (β1) avec le plan d'appui (7) qui n'est pas inférieur à 30°, le deuxième plan (Pβ) formant aussi avec le premier plan (Pα) un troisième angle minimum (αβ) qui n'est pas inférieur à 30°.

8. Le dispositif selon la revendication 7, **caractérisé :**

**en ce que** le premier plan (Pα) et le deuxième plan (Pβ) forment le premier angle minimum (α1) et le deuxième angle minimum (β1) respectivement sur des côtés opposés par rapport à un plan perpendiculaire à la direction nominale des fibres (N) ; et/ou

**en ce que** le premier angle minimum (α1) est égal au deuxième angle minimum (β1) ; et/ou **en ce que** le premier angle minimum (α1) et le deuxième angle minimum (β1) sont compris entre 30° et 60° ; ou en ce que le premier plan (Pα) est orthogonal au plan d'appui (7), et en ce que le deuxième angle minimum (β1) est compris entre 30° et 60°.

9. Le dispositif selon la revendication 8, **caractérisé en ce que** le premier angle minimum (α1) est égal au deuxième angle minimum (β1) et **en ce que** les moyens d'émission (8) et les moyens de détection (9) sont les uns en face des autres de manière à définir aussi au moins un troisième plan (Pγ) pour la propagation des faisceaux de micro-ondes des moyens d'émission (8) aux moyens de détection (9), distinct du premier plan (Pα) et du deuxième plan (Pβ) et passant à travers la zone opérationnelle (10) ; le troisième plan (Pγ) étant orthogonal au plan d'appui (7) et coupant le plan d'appui (7) le long d'une troisième ligne transversale à la direction nominale des fibres (N) et parallèle à la première ligne transversale ; le troisième plan (Pγ) formant avec le premier plan (Pα) ainsi qu'avec le deuxième plan (Pβ) un quatrième angle minimum qui n'est pas inférieur à 30°.

10. Le dispositif selon l'une quelconque des revendications de 7 à 9, **caractérisé en ce que** la première ligne transversale est orthogonale à l'axe longitudinal.

11. Le dispositif selon l'une quelconque des revendications de 7 à 10, **caractérisé en ce que** alternativement :

   - les moyens d'émission (8) comprennent un émetteur de micro-ondes pour chaque plan de propagation et les moyens de détection (9) comprennent un détecteur de micro-ondes pour chaque plan de propagation ; ou
   - les moyens d'émission (8) comprennent un seul émetteur de micro-ondes pour tous les plans de propagation et les moyens de détection (9) comprennent un détecteur de micro-ondes pour chaque plan de propagation ; ou
   - les moyens de détection (9) comprennent un seul détecteur de micro-ondes pour tous les plans de propagation et les moyens d'émission (8) comprennent un émetteur de micro-ondes pour chaque plan de propagation.

12. Le dispositif selon la revendication 11, **caractérisé en ce que** chaque émetteur comprend une ou plusieurs antennes émettrices (13) qui sont alignées dans une direction parallèle à la première ligne transversale, et/ou **en ce que** chaque émetteur comprend une ou plusieurs antennes émettrices (13) qui sont alignées dans une direction parallèle à la première ligne transversale et chaque récepteur comprend une ou plusieurs antennes réceptrices qui sont alignées dans une direction parallèle à la première ligne transversale.

13. Un appareil pour estimer la projection sur un plan de référence (XZ) de la direction d'extension des fibres (L) d'une portion d'une planche de bois (1), la planche de bois (1) ayant une direction nominale des fibres (N) qui est parallèle à son axe d'extension principal et une première face (2) qui est elle aussi parallèle à l'axe d'extension principal ;
   l'appareil (4) comprenant :

   au moins un dispositif (5) réalisé selon l'une quelconque des revendications de 7 à 12 ; et
   au moins une unité électronique de contrôle et de traitement (14) opérationnellement reliée au moins aux moyens d'émission (8) et aux moyens de détection (9) et programmée pour traiter ce qui a été détecté par les moyens de détection (9) afin d'estimer ladite projection.

14. L'appareil selon la revendication 13, **caractérisé en ce que** l'unité électronique de contrôle et de traitement (14) est programmée pour mettre en oeuvre au moins les phases d'émission, de détection, de détermination et de traitement du procédé selon l'une quelconque des revendications de 1 à 6.

FIG. 1

FIG. 2

FIG. 3

FIG. 4

FIG. 5

FIG. 6

FIG. 7

FIG. 8

FIG. 9

## REFERENCES CITED IN THE DESCRIPTION

### Patent documents cited in the description

- CA 2046895
- US 6842010 B
- US 4500835 A
- US 6859046 B
- US 2003222657 A

### Non-patent literature cited in the description

- **BOGOSANOVIC M ; ANBUKY A.A ; EMMS G.W.** Microwave measurement of wood in principal directions, Sensors. IEEE, 2009, 252-256
- **BOGOSANOVIC M ; ANBUKY A.A ; EMMS G.W.** Overview and comparison of microwave noncontact wood measurement techniques. *Journal of Wood Science,* 2010, vol. 56 (5), 357-365
- **BOGOSANOVIC M ; ANBUKY A.A ; EMMS G.W.** Microwave measurement of wood anisotropy, Sensors Applications Symposium (SAS). IEEE, 2011, 262-267
- **HEIKILLA S ; JAKKULA P ; TIURI M.** Microwave methods for strength grading of timber and for automatic edging of boards. *Proceedings of the 12th European Microwave Conference,* 1982, 599-603
- **JAMES W.L ; YEN YH ; KING R.J.** A microwave method for measuring moisture content, density and grain angle of wood. *Forest Products Laboratory, Research Note FPL-0250,* 1985
- **LEICESTER R.H ; SEATH C.A.** Application of microwave scanners for stress grading. *Conference Proceedings - International Wood Engineering Conference,* 1996, vol. 2, 435-440
- **MALIK S.A ; GHODOGOANKAR D.K ; HAMBALY A.M ; MAJID W.M ; NURUDDIN M.F.** Measurement of wood grain angle using free-space microwave system in 8-12GHz frequency range. *Proceedings of the Asian Conference on Sensors and the International Conference on New Techniques in Pharmaceutical and Biomedical Research,* 2005, 213-218
- **MARTIN P ; COLLET R ; BARTHELEMY P ; ROUSSY G.** Evaluation of wood characteristics: Internal scanning of the material by microwaves. *Wood Science and Technology,* 1987, vol. 21/4, 361-371
- **SCHAJER G.S ; ORHAN F.B.** Microwave non-destructive testing of wood and similar orthotropic materials. *Subsurface Sensing Technologies and Applications,* 2005, vol. 6, 293-313
- **SHEN J ; SCHAJER G.S ; PARKER R.** Theory and practice in measuring wood grain angle using microwaves. *IEEE Trans Instrum. Meas.,* 1994, vol. 43, 803-809
- **BOGOSANOVIC M ; ANBUKY A.A ; EMMS G.W.** Overview and comparison of microwave noncontact wood measurement technique. *Journal of Wood Science,* 2010, vol. 56 (5), 357-365